(19) **Europäisches Patentamt / European Patent Office / Office européen des brevets**

(11) **EP 3 967 743 B1**

(12) **FASCICULE DE BREVET EUROPEEN**

(45) Date de publication et mention de la délivrance du brevet:
**22.11.2023 Bulletin 2023/47**

(21) Numéro de dépôt: **21195782.4**

(22) Date de dépôt: **09.09.2021**

(51) Classification Internationale des Brevets (IPC):
***C12M 1/34*** *(2006.01)*

(52) Classification Coopérative des Brevets (CPC):
**C12M 41/00**

(54) **KIT D'ANALYSE ET DE SUIVI D'UN PARAMETRE, AU MOYEN D'UN CAPTEUR MOBILE, D'UNE REACTION CHIMIQUE OU BIOCHIMIQUE OU BIOLOGIQUE DANS UNE ENCEINTE REACTIONNELLE**

KIT ZUR ANALYSE UND ÜBERWACHUNG EINES PARAMETERS EINER CHEMISCHEN ODER BIOCHEMISCHEN ODER BIOLOGISCHEN REAKTION IN EINER REAKTIONSKAMMER MITHILFE EINES MOBILEN SENSORS

KIT FOR ANALYSING AND MONITORING A PARAMETER, BY MEANS OF A MOBILE SENSOR, A CHEMICAL OR BIOCHEMICAL OR BIOLOGICAL REACTION IN A REACTION CHAMBER

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.09.2020 FR 2009160**

(43) Date de publication de la demande:
**16.03.2022 Bulletin 2022/11**

(73) Titulaire: **Commissariat à l'énergie atomique et aux énergies alternatives**
**75015 Paris (FR)**

(72) Inventeurs:
• **CAROFF, Tristan**
  **38054 GRENOBLE CEDEX 9 (FR)**
• **BRULAIS, Sébastien**
  **38054 GRENOBLE CEDEX 9 (FR)**
• **GAUROY, Martin**
  **38054 GRENOBLE CEDEX 9 (FR)**
• **NIEF, Nadège**
  **38054 GRENOBLE CEDEX 9 (FR)**

(74) Mandataire: **Brevalex**
**56, Boulevard de l'Embouchure**
**B.P. 27519**
**31075 Toulouse Cedex 2 (FR)**

(56) Documents cités:
**EP-A2- 2 065 701      WO-A1-2012/130334**
**FR-A1- 3 053 165**

**Description**

**DOMAINE TECHNIQUE**

L'invention se rapporte aux domaines de l'industrie pharmaceutique, de la cosmétique, de la production biomasse, de l'agroalimentaire.

En particulier, l'invention concerne un kit d'analyse et de suivi d'au moins un paramètre d'une réaction chimique ou biochimique ou biologique dans une enceinte réactionnelle. Notamment, le kit de suivi est configuré pour permettre la mesure d'au moins un paramètre d'une réaction, par exemple une réaction biologique, et plus particulièrement de dresser une cartographie dans l'enceinte réactionnelle dudit paramètre afin de suivre l'homogénéité de la réaction.

**ÉTAT DE LA TECHNIQUE ANTÉRIEURE**

La multiplication ou la culture des micro-organismes est largement mise en oeuvre dans l'industrie pharmaceutique, la cosmétologie ou encore l'industrie agroalimentaire pour la production de biomasse, pour la production d'un métabolite ou encore la bioconversion d'une molécule d'intérêt, mais également la vinification et le brassage.

Cette multiplication est avantageusement exécutée dans un bioréacteur qui permet d'exercer un contrôle des conditions de culture, et plus particulièrement des paramètres réactionnels tels que la température, le pH, le niveau d'oxygène, le taux de croissance de la biomasse...

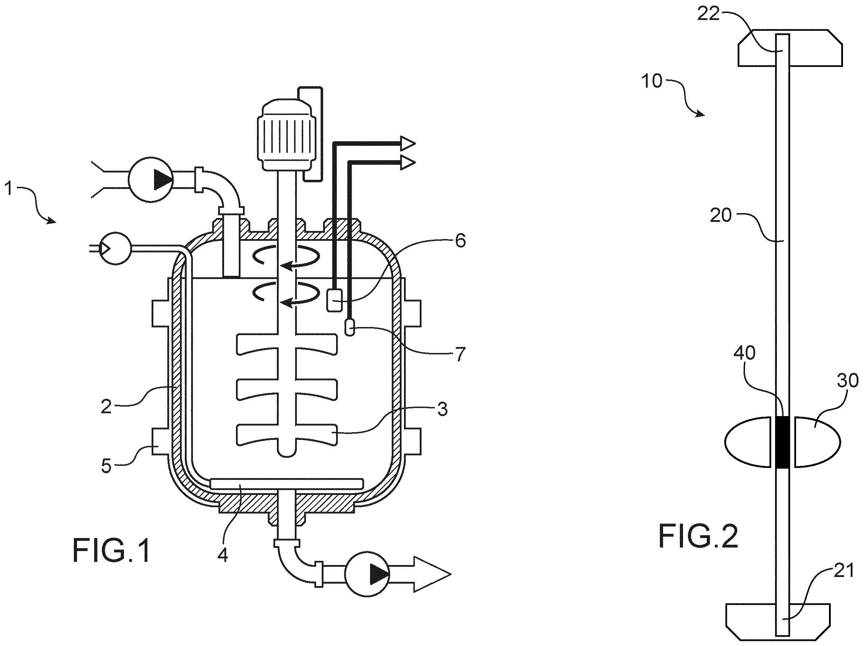

Un bioréacteur, tel qu'illustré à la figure 1, comprend une enceinte réactive 2 dans laquelle sont disposés un agitateur 3 et un aérateur 4 destinés à, respectivement, homogénéiser et oxygéner le milieu réactionnel. Le bioréacteur comprend en outre une enveloppe thermique 5 qui permet d'imposer une thermalisation au milieu réactionnel. Le bioréacteur 1 est également pourvu de capteurs, par exemple des capteurs de température, de pH, d'oxygène dissous, afin d'assurer un suivi de l'état de la culture, et, le cas échéant, ajuster, de préférence en temps réel, les conditions de culture.

Selon une première alternative, ces capteurs 6, 7, tel qu'illustré à la figure 1, peuvent être montés de manière fixe sur des tiges plongeant dans le milieu réactionnel.

Cependant, cette première alternative n'est pas satisfaisante.

En effet, les capteurs maintenus fixes ne sondent le milieu réactionnel que dans leur proche environnement, et restent, par voie conséquence, insensibles à toute inhomogénéité dudit milieu. Les données collectées par ces capteurs ne permettent toutefois pas de procéder à un ajustement optimal des paramètres réactionnels.

Ainsi, afin de mieux rendre compte de l'inhomogénéité du milieu réactionnel, il est possible, selon une deuxième alternative, de considérer un capteur mobile. Un tel capteur est notamment logé dans une capsule, par exemple une capsule sphérique. Plongée dans le milieu réactionnel, la capsule suit le ou les courants imposés par l'agitateur et est ainsi exposée à différentes zones du milieu réactionnel.

Parmi les capsules mobiles aujourd'hui disponibles pour le suivi de l'état de la culture, on peut citer celle développée par SensOsphere - Fraunhofer et décrite dans le document [1] cité à la fin de la description. Cette capsule comprend notamment un capteur de mesure de pH, un capteur de mesure de température. Elle est outre dotée d'une puce de géolocalisation qui permet de suivre en temps réel son déplacement dans le bioréacteur, ainsi qu'un moyen de transmission de données sans fil.

Aussi, afin d'établir un profil statistique des paramètres réactionnels le plus représentatif possible du milieu réactionnel, il peut être considéré de mettre en oeuvre une pluralité de ces capsules. Ces dernières peuvent alors collecter de manière collective une grande quantité de données. Toutefois, la transmission de ces données, à travers le milieu réactionnel, vers un récepteur reste compliquée à mettre en oeuvre.

Une autre capsule, développée par la société SmartINST et décrite dans les documents [2] et [3] cités à la fin de la description, peut également être mise en oeuvre. Cette capsule est dotée d'une pluralité de capteurs et autorise une transmission par radiofréquence des données collectées. Néanmoins, cette capsule, dépourvue de moyens de géolocalisation, ne permet pas de dresser une cartographie des données collectées, et n'offre donc pas la possibilité d'ajuster les paramètres réactionnels de manière optimale.

Le document [4], cité à la fin de la description, décrit une autre capsule développée par la société FreeSense. Cette dernière pourvue d'un capteur de mesure de pH et d'un capteur de mesure de température est dédiée au suivi de la fermentation dans le milieu réactionnel. La capsule, qui ne comprend pas de moyens de transmission de données, interdit toute optimisation des paramètres réactionnels en temps réel. Des moyens de géolocalisation, formés par un accéléromètre et un capteur de pression intégrés à la capsule, permettent de localiser cette dernière. Ces moyens de géolocalisation nécessitent toutefois la mise en oeuvre d'algorithmes complexes, et plus particulièrement d'un filtrage de Kalman, très exigeant en termes de ressources de calcul. Par ailleurs, ces moyens de géolocalisation doivent être très régulièrement calibrés.

Enfin, une autre capsule décrite dans le document [5] cité à la fin de la description peut également être mise en oeuvre. Cette capsule permet le suivi du pH, de la température et de l'aération. Néanmoins, cette dernière est dépourvue de moyens de transmission en temps réel des données collectées, et de moyens de géolocalisation. WO2021130334 divulgue un bioréacteur avec un système comprenant un capteur sur une corde reliée a deux poulies pour positionner le capteur dans le bioréacteur.

De manière générale, les capsules décrites ci-avant, dans la mesure où leur déplacement dans le bioréacteur est libre, peuvent entrer en contact avec les parois du bioréacteur ou des pales de l'agitateur, et risquent par conséquent d'être endommagées.

Par ailleurs, le parcours des capsules dans le milieu réactionnel reste aléatoire, et ne permet pas de couvrir toute l'étendue du bioréacteur.

Ainsi, un but de la présente invention est de proposer un système de suivi d'une réaction dans une enceinte réactive permettant de limiter les risques d'endommagement de la cellule.

Un autre but de la présente invention est de proposer un système de suivi d'une réaction dans une enceinte réactive permettant une localisation simplifiée au regard des cellules connues de l'état de la technique.

Un autre but de la présente invention est de proposer un système de suivi de réaction dans une enceinte réactive permettant un transfert simple des données et en temps réel. Un autre but de la présente invention est de proposer un système de suivi de réaction dans une enceinte réactive permettant de sonder l'intégralité du volume de ladite enceinte.

## EXPOSÉ DE L'INVENTION

Les buts de l'invention sont, au moins en partie, atteints par un kit d'analyse et de suivi d'au moins un paramètre d'une réaction chimique ou biochimique ou biologique dans une enceinte réactionnelle, le kit comprenant :

- un tube creux qui s'étend selon deux extrémités dites, respectivement, première extrémité et deuxième extrémité, destinées à être fixées sur des parois de l'enceinte réactionnelle ;
- une capsule sonde en liaison glissière avec le tube creux, la capsule sonde comprend en outre au moins un capteur configuré pour mesurer l'au moins un paramètre, et des moyens de transmission des données susceptibles d'être mesurées par l'au moins un capteur ;
- un piston magnétique logé à l'intérieur du tube creux, coopérant de manière magnétique avec la capsule sonde, de sorte qu'un déplacement dudit piston magnétique impose un déplacement à la capsule sonde ;
- un moyen de commande agencé pour commander le déplacement du piston magnétique ;
- des moyens de réception des données susceptibles d'être transmises par les moyens de transmission.

Selon un mode de mise en oeuvre, la capsule sonde comprend un canal de guidage traversé par le tube creux.

Selon un mode de mise en oeuvre, le moyen de commande comprend des moyens d'indexation permettant la détermination de la position de la capsule sonde le long du tube creux.

Selon un mode de mise en oeuvre, le moyen de commande comprend des moyens pneumatiques agencés pour injecter selon l'une et/ou l'autre de la première extrémité et de la deuxième extrémité un fluide permettant d'imposer un déplacement au piston magnétique aimanté.

Selon un mode de mise en oeuvre, les moyens pneumatiques comprennent un réservoir muni d'un piston mobile dont le mouvement contrôlé par un actionneur, notamment un actionneur linéaire, induit l'injection de fluide selon l'une et/ou l'autre de la première extrémité et de la deuxième extrémité.

Selon un mode de mise en oeuvre, le moyen de commande comprend un ou plusieurs câbles agencés pour guider le piston magnétique le long du tube creux.

Selon un mode de mise en oeuvre, le piston magnétique comprend un aimant ou un électroaimant destiné à assurer la coopération magnétique entre le piston magnétique et la capsule sonde.

Selon un mode de mise en oeuvre, le piston magnétique comprend en outre les moyens de réception de sorte que la transmission des données susceptibles d'être mesurées par l'au moins un capteur soient transmises de la capsule vers le piston magnétique selon une transmission radio à travers le tube creux.

Selon un mode de mise en oeuvre, la capsule et le piston magnétique comprennent chacun des moyens d'induction coopérant entre eux et agencés pour alimenter en énergie ladite sonde.

Selon un mode de mise en oeuvre, la capsule comprend une batterie destinée à assurer l'apport en énergie nécessaire au fonctionnement de l'au moins une sonde et des moyens de transmission.

Selon un mode de mise en oeuvre, les moyens de réception sont logés dans un module externe destiné à être disposé hors de l'enceinte réactionnelle et contre la paroi de ladite enceinte.

Selon un mode de mise en oeuvre, ledit kit comprend des moyens de charge par induction de la batterie.

Selon un mode de mise en oeuvre, les moyens de charge comprennent une bobine primaire logée dans le module externe et une bobine secondaire logée dans la capsule. Selon un mode de mise en oeuvre, le tube creux forme un hélicoïde, notamment un hélicoïde à spires jointives, et est configuré pour être étiré et s'étendre de manière essentiellement linéaire entre sa première extrémité et sa deuxième extrémité.

Selon un mode de mise en oeuvre, l'au moins un capteur comprend au moins un des éléments choisis parmi : un capteur de mesure de pH, un capteur de mesure de température, un capteur de mesure d'une quantité d'oxygène dissous, un capteur de mesure d'une quantité de dioxyde de carbone.

L'invention concerne également un réacteur qui comprend :

- une enceinte réactionnelle qui comprend une enveloppe délimitant un volume interne ;
- le kit selon la présente invention.

Selon un mode de mise en oeuvre, le tube creux est disposé dans le volume interne de l'enveloppe, et est fixé à cette dernière par ces deux extrémités de sorte que deux ouvertures du tube creux associées à chacune des extrémités débouchent vers l'extérieur de l'enveloppe.

Selon un mode de mise en oeuvre, le module externe du kit est agencé de sorte que lorsque la capsule est en butée contre ladite enveloppe au niveau de la première extrémité, la transmission de données s'effectue au travers de ladite enveloppe.

Selon un mode de mise en oeuvre, le module externe est fixé audit réacteur au niveau de la première extrémité du tube creux.

Selon un mode de mise en oeuvre, l'enveloppe est formée par un contenant gonflable qui lorsqu'il est gonflé étire le tube creux du kit de manière à étendre ce dernier de manière linéaire entre sa première extrémité et sa deuxième extrémité.

L'invention concerne également un procédé de suivi d'une réaction chimique, biochimique ou biologique mis en oeuvre dans un réacteur selon la présente invention, et qui comprend les étapes suivantes :

a) une étape de collecte d'un ensemble de mesures d'au moins un paramètre avec l'au moins un capteur à différentes positions de la capsule le long du tube creux ;
b) une étape d'ajustement de conditions réactionnelles sur la base de l'ensemble des mesures collectées à l'étape a).

## BRÈVE DESCRIPTION DES DESSINS

D'autres caractéristiques et avantages apparaîtront dans la description qui va suivre d'un kit de suivi d'un paramètre d'une réaction selon l'invention, donnés à titre d'exemples non limitatifs, en référence aux dessins annexés dans lesquels :

[Fig. 1] est une représentation schématique d'une enceinte réactionnelle, connue de l'état de la technique, pourvue de capteurs de suivi de l'état d'une réaction ;

[Fig. 2] est une représentation schématique d'un kit d'analyse et de suivi selon la présente invention ;

[Fig. 3] est une représentation schématique de l'agencement d'aimants au sein du piston magnétique et de la capsule selon la présente invention ;

[Fig. 4] est une représentation schématique du kit selon la présente invention, notamment la figure 4 représente le kit pourvu de moyens de guidage mécaniques selon une première variante ;

[Fig. 5] est une représentation schématique du kit selon la présente invention, notamment la figure 5 représente le kit pourvu de moyens de guidage pneumatiques selon une deuxième variante ;

[Fig. 6] est une représentation schématique de la capsule et du piston magnétique selon un premier mode de réalisation de la présente invention, notamment la figure 6 illustre le mode de communication entre les moyens de transmission et les moyens de réception, et le mode d'alimentation de la capsule et du piston aimanté ;

[Fig. 7] est une représentation schématique de la capsule et du piston magnétique selon un deuxième mode de réalisation de la présente invention, notamment la figure 7 illustre l'agencement d'un module externe destiné à assurer la charge d'une batterie disposée dans la cellule et la réception de données collectées par le ou les capteurs ;

[Fig. 8] est une vue selon un plan de coupe et en perspective d'un réacteur pourvu du kit selon la présente invention ;

[Fig. 9] est une représentation schématique du réacteur mettant en oeuvre le kit pourvu de moyens de commande réalisés selon la première variante ;

[Fig. 10] est une représentation schématique du réacteur mettant en oeuvre le kit pourvu de moyens de commande selon la première variante et pourvu d'un unique câble couplé à une capsule/sonde flottante ;

[Fig. 11] est une représentation schématique du réacteur mettant en oeuvre le kit pourvu de moyens de commande réalisés selon la deuxième variante ;

[Fig. 12] est une représentation schématique d'une enveloppe gonflable dans un état dégonflé et qui comprend un tube creux en forme d'hélicoïde ;

[Fig. 13] est une représentation schématique de l'enveloppe de la figure 12, dans un état gonflé.

## EXPOSÉ DÉTAILLÉ DE MODES DE RÉALISATION PARTICULIERS

La présente invention concerne un kit d'analyse et de suivi d'au moins un paramètre d'une réaction chimique ou biochimique ou biologique dans une enceinte réactionnelle. Le kit comprend notamment un tube creux destiné à être fixé par ses extrémités à l'enceinte réactionnelle. Le kit est également doté d'une capsule, pourvue d'un ou plusieurs capteurs, et susceptible d'être guidée le long du tube creux. Ce guidage est par exemple exécuté au moyen d'un piston magnétique aimanté disposé dans le tube creux et commandé par des moyens de guidage.

Le kit comprend enfin des moyens de réception des données susceptibles d'être collectées par l'au moins un capteur.

Selon la présente invention, le tube creux impose un parcours prédéterminé à la capsule, et permet ainsi de maintenir à distance ladite capsule de tout organe mobile, tel qu'un agitateur. La capsule est ainsi préservée de tout endommagement.

Par ailleurs, le tube creux peut s'étendre sur toute la profondeur du milieu réactionnel et ainsi permette des mesures, par le ou les capteurs, sur toute la profondeur dudit milieu. Plus particulièrement, la mise en oeuvre du tube creux comme guide permet de sonder des zones du milieu réactionnel qui ne le seraient pas avec une capsule libre plongée dans ledit milieu.

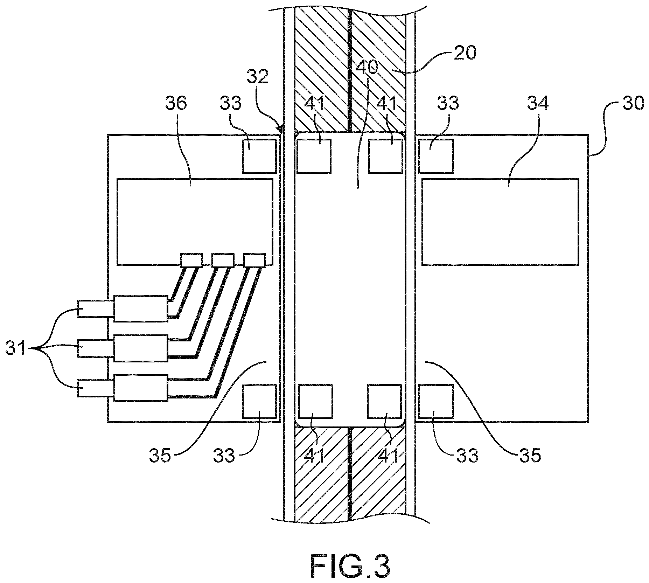

Le kit 10 selon la présente invention est décrit en relation avec les figures 2 et 3.

Le kit 10 est notamment pourvu d'un tube creux 20 qui comprend deux extrémités dites, respectivement, première extrémité 21 et deuxième extrémité 22.

Le tube creux 20 peut comprendre un matériau polymère et présenter un diamètre compris entre 1 cm et 5 cm.

Le kit 10 comprend également une capsule 30 pourvu d'un ou de plusieurs capteurs 31 (figure 3), destinés à collecter des données d'un milieu réactionnel dans lequel la capsule 30 est susceptible d'être plongée. À cet égard, le milieu réactionnel peut être un milieu liquide dans lequel se déroule une réaction chimique, biochimique ou biologique. Notamment, les données collectées par le ou les capteurs peuvent être relatives aux conditions expérimentales imposées au milieu réactionnel. Ces dernières peuvent être caractéristiques de l'acidité, de l'agitation, de la température. Les données collectées peuvent également être relatives à l'avancement de la réaction ou des phénomènes mis en jeu dans le milieu réactionnel.

À titre d'exemple, les capteurs 31 peuvent comprendre au moins un des éléments choisis parmi : un capteur de mesure de pH, un capteur de mesure de température, un capteur de mesure d'une quantité d'oxygène dissous, un capteur de mesure d'une quantité de dioxyde de carbone. L'invention n'est pas limitée à ces seuls capteurs, et l'homme du métier, en fonction des paramètres réactionnels à suivre, pourra mettre en oeuvre tout autre capteur.

La capsule 30 selon la présente invention est en liaison glissière avec le tube creux 20 de manière à pouvoir être guidée le long dudit tube creux 20. La liaison glissière peut, selon un mode de réalisation avantageux, être mise en oeuvre au moyen d'un canal de guidage 32 ménagé dans la capsule 30 et traversé par le tube creux 20. Il est entendu que le canal de guidage traverse de part en part la capsule 30.

Un piston magnétique 40 (figures 2 et 3) est également logé à l'intérieur du tube creux 20. Le piston magnétique 40 est d'une forme complémentaire, par exemple cylindrique, à la forme d'une surface intérieure du tube creux.

Ce piston magnétique 40 coopère de manière magnétique avec la capsule 30, de sorte qu'un déplacement dudit piston magnétique, dans le tube creux, impose un déplacement identique à la capsule 30 le long dudit tube. À cet égard, le piston magnétique 40 peut comprendre un ou plusieurs aimants ou électroaimants 41, interagissant avec d'autres aimants 33 logés dans la capsule 30.

Le kit 10 comprend également un moyen de commande 50 agencé pour commander le déplacement du piston magnétique 40 le long du tube creux 20 et ainsi entraîner la capsule 30. Ce moyen de commande, décrit en détails dans la suite de la présente invention, peut par ailleurs comprendre des moyens d'indexation permettant la détermination de la position de la capsule sonde le long du tube creux.

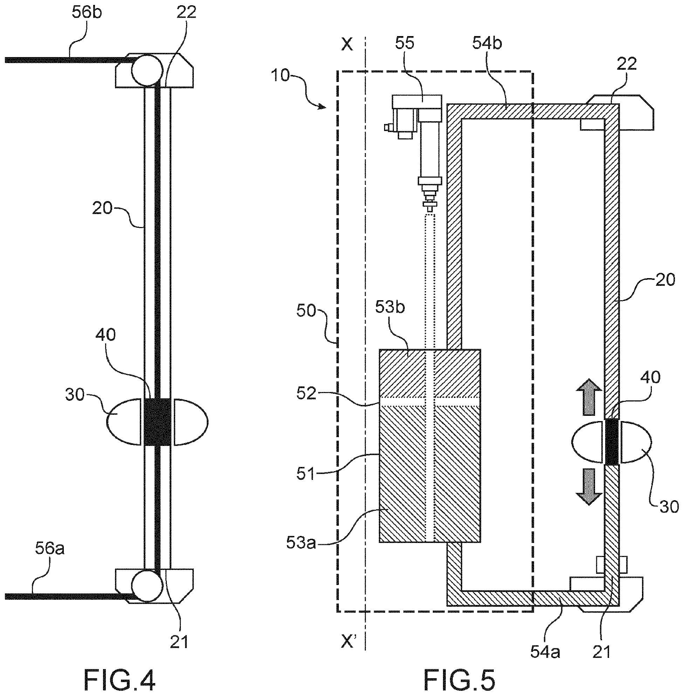

Selon une première variante illustrée à la figure 4, le moyen de commande peut comprendre des moyens mécaniques. En particulier, ces moyens mécaniques peuvent comprendre des câbles dits, premier câble 56a et deuxième câble 56b, fixés au piston magnétique 40. En particulier, le premier câble 56a est agencé pour, lorsqu'il est en tension, attirer le piston aimanté 40 vers la première extrémité 21. De manière équivalente, le deuxième câble 56b est agencé pour, lorsqu'il est en tension, attirer le piston aimanté 40 vers la deuxième extrémité 22. Plus particulièrement, le premier câble 56a est fixé au piston aimanté 40 et sort du tube creux au niveau de la première extrémité 21. De manière équivalente, le deuxième câble 56b est fixé au piston aimanté 40 et sort du tube creux au niveau de la deuxième extrémité 22. Le moyen de commande selon cette première variante peut également comprendre un moteur, associé à un ensemble de poulies et/ou de treuils, agencé pour exercer une tension sur l'un et l'autre du premier câble 56a et du deuxième câble 56b. Selon une alternative à cette deuxième variante, un unique câble, par exemple le premier câble 56a peut être mis en oeuvre, en étant par exemple couplée à une capsule flottante.

Selon une deuxième variante illustrée à la figure 5, le moyen de commande peut comprendre des moyens pneumatiques. En particulier, ces moyens pneumatiques sont agencés pour injecter, selon l'une et/ou l'autre de la première extrémité 21 et de la deuxième extrémité 22, un fluide permettant d'imposer un déplacement au piston magnétique 40.

En particulier, le moyen de commande pneumatique comprend un réservoir 51 pourvu d'un piston mobile 52 mobile selon une direction d'élongation XX' dudit réservoir. Le piston mobile 52 sépare notamment le volume du réservoir 51 en deux sections de volume variable dites, respectivement, première section 53a et deuxième section 53b, et remplies chacune d'un fluide, et plus particulièrement d'un fluide incompressible, par exemple de l'eau. Le fluide peut également comprendre une huile, par exemple une huile chargée en particules métalliques permettant de la rendre électriquement conductrice.

La première section 53a est connectée fluidiquement au tube creux 20 par la première extrémité 21 au moyen d'un premier conduit 54a. De manière équivalente, la deuxième section 53b est connectée fluidiquement au tube creux 20 par la deuxième extrémité 22 au moyen d'un deuxième conduit 54b.

Ainsi, en fonctionnement, un déplacement du piston mobile 52, conduisant à une réduction du volume de la première section 53a, est accompagné d'une injection de fluide de la première section 53a vers la première extrémité 21 du tube creux 20. Lors de cette injection, le piston magnétique 40 est poussé par ledit fluide en direction de la deuxième extrémité 22 et entraine avec lui, par interaction magnétique, la capsule 30. Le mouvement du piston magnétique 40 en direction de la deuxième extrémité 22 chasse le fluide compris dans le tube creux 20 entre ledit piston magnétique 40 et la deuxième extrémité 22 vers la deuxième section 53b.

De manière équivalente, un déplacement du piston mobile 52 conduisant à une réduction du volume de la deuxième section 53b, est accompagné d'une injection de fluide de la deuxième section 53b vers la deuxième extrémité 22 du tube creux 20. Lors de cette injection, le piston magnétique 40 est poussé par ledit fluide en direction de la première extrémité 21 et entraine avec lui, par interaction magnétique, la capsule 30. Le mouvement du piston magnétique 40 en direction de la première extrémité 21 chasse le fluide compris dans le tube creux 20 entre ledit piston magnétique 40 et la première extrémité 21 vers la première section 53a.

Selon cette deuxième variante, le mouvement du piston mobile 52 peut être mis en oeuvre par un moyen d'entrainement, par exemple un actionneur linéaire 55. Le moyen d'entrainement peut à cet égard comprendre les moyens d'indexation permettant de déterminer la position de la capsule sur le tube creux.

Le kit 10 selon la présente invention comprend également des moyens de transmission 34 des données susceptibles d'être mesurées par le ou les capteurs 31. En particulier, les moyens de transmission 34, pourvus d'une électronique adaptée, peuvent être logés dans la capsule 30.

Les moyens de transmission 34 sont par ailleurs adaptés pour coopérer avec des moyens de réception 60. Notamment, les moyens de transmission 34 sont adaptés pour transmettre les données collectées par le ou les capteurs 31 vers les moyens de réception 60. Cette transmission des données peut en particulier être effectuée selon un mode de communication sans fil, plus particulièrement une communication radio (par exemple en utilisant la bande 125 kHz ou la bande 1356 MHz).

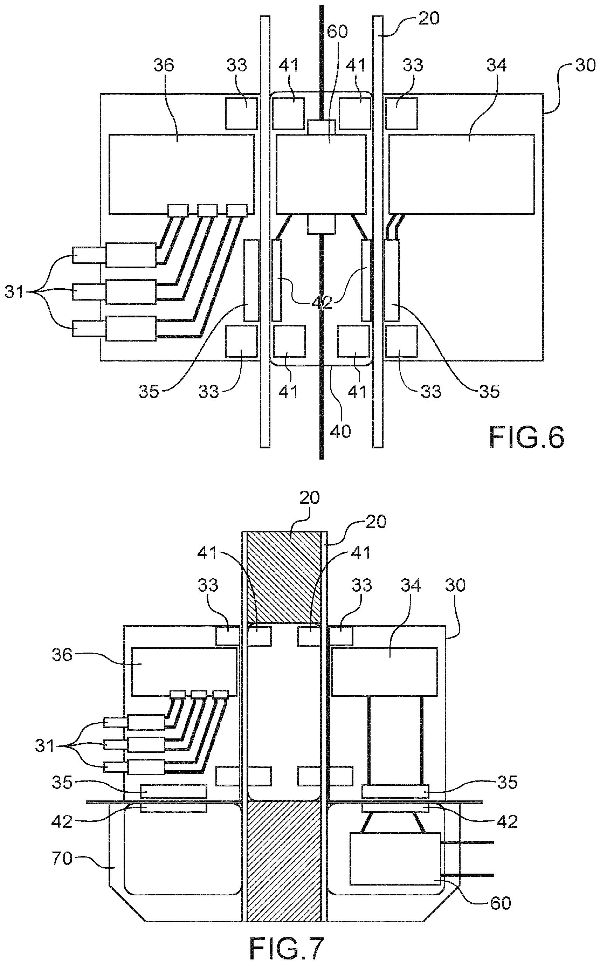

Selon un premier mode de réalisation de la présente invention illustré à la figure 6, les moyens de réception 60 sont logés dans le piston magnétique 40. Ainsi, la communication sans fil entre les moyens de transmission 34 et les moyens de réception 60 s'effectue au travers de la paroi du tube creux 20. De manière particulièrement avantageuse, le piston aimanté 40 peut être connecté à un câble d'alimentation électrique destiné à alimenter les moyens de réception 60. Cette alimentation électrique peut également être mise à profit pour l'apport de l'énergie nécessaire au fonctionnement des capteurs, de l'électronique 36 qui leur est associée et des moyens de transmission. L'apport de cette énergie, à partir du piston aimanté 40, peut mettre en oeuvre des moyens d'induction formés par des bobines primaires 42 et des bobines secondaires 35 portées respectivement par le piston aimanté 40 et la capsule 30. La communication radio entre les moyens de transmission 34 et les moyens de réception 60 peut, à cet égard, être assurée par les bobines primaires 42 et les bobines secondaires 35.

Cet agencement ne nécessite pas la mise en oeuvre d'une batterie ou d'un accumulateur pour le fonctionnement des différents éléments portés par la capsule 30.

Par ailleurs, la communication radio entre les moyens de transmission 34 et les moyens de réception 60 au travers de la paroi du tube creux 20 n'est que peu ou pas écrantée.

Le dimensionnement des bobines primaires et des bobines secondaires doit permettre de répondre aux besoins de puissance d'un tel agencement selon le premier mode de réalisation. Afin de mieux les apprécier, l'exemple suivant, non limitatif, évalue les besoins et les consommations des différents éléments formant le kit selon la présente invention.

À cet égard, la capsule 30 peut comprendre :

- un capteur de pH dont la consommation est de l'ordre de 300 $\mu$W ;
- un ou plusieurs capteurs de température dont la consommation est de l'ordre de 20 $\mu$W ;
- un capteur de dioxygène dissous (DO) dont la consommation est de l'ordre de 100 $\mu$W ;

- une électronique associée aux capteurs dont la consommation est de l'ordre de 100 μW.

L'unité de gestion de la communication radio est, selon cet exemple, alimentée par alimentée par les moyens de réception.

Ainsi, les besoins en termes de puissance totale sont de l'ordre de 550 μW.

Une télé-alimentation mettant en oeuvre un protocole Qi (ex BQ51003), typiquement en mesure de délivrer une puissance continue de 2,5 W, permet de répondre aux besoins énergétiques du kit selon la présente invention.

Ce premier mode de réalisation peut avantageusement être combiné avec la première variante. En effet, le ou les câbles mis en oeuvre dans le cadre de la première variante peuvent assurer l'apport en énergie.

La figure 7 illustre un deuxième mode de réalisation de la présente invention. Selon ce mode de réalisation, le kit 10 comprend un module externe 70 destiné à être disposé à la base du tube creux, par exemple au niveau de la première extrémité. Le module externe 70 comprend notamment les moyens de réception 60. En particulier, le module externe 70 est agencé de manière à permettre une communication radio entre les moyens de réception 60 et les moyens de transmission 34 lorsque la capsule 30 est en fin de course au niveau de la première extrémité 21 du tube creux 20. La capsule 30 peut par ailleurs comprendre une batterie ou un accumulateur fournissant l'énergie nécessaire au fonctionnement des capteurs, de l'électronique 36 qui leur est associée et des moyens de transmission 34. À l'instar du premier mode de réalisation, des moyens d'induction formés par les bobines primaires 42 et les bobines secondaires 35 sont également mis en oeuvre. En particulier, les bobines secondaires 35 sont portées par la capsule 30 tandis que les bobines primaires le sont par le module externe 70. Ces bobines primaires et secondaires sont par ailleurs agencées pour permettre la charge ou la recharge de la batterie logée dans la capsule 30 lorsque la capsule 30 est en fin de course au niveau de la première extrémité 21 du tube creux 20. La communication radio entre les moyens de transmission 34 et les moyens de réception 60 peut également être assurée par les bobines primaires 42 et les bobines secondaires 35. Ainsi, la batterie ou l'accumulateur permet un fonctionnement autonome de la capsule 30 qui peut collecter des données au moyen des capteurs 31 qu'elle porte lors de déplacements de va et vient le long du tube creux. Ces phases d'arrêt au niveau de la première extrémité, en position dite d'accostage, peuvent intervenir afin de transmettre les données collectées par les capteurs aux moyens de réception 60 et recharger la batterie. Le module externe 70 peut être disposé hors de l'enceinte réactionnelle, mais au contact de cette dernière. Ainsi, la communication entre les moyens de réception 60 et les moyens de transmission 34 se font au travers de la paroi de l'enceinte réactionnelle.

Ce deuxième mode de réalisation est avantageusement associé des moyens de commande selon la deuxième variante.

Le dimensionnement des bobines primaires et des bobines secondaires doit permettre de répondre aux besoins de puissance d'un tel agencement selon le deuxième mode de réalisation. Afin de mieux les apprécier, l'exemple suivant, non limitatif, évalue les besoins et les consommations des différents éléments formant le kit selon la présente invention. La capsule considérée reprend pour l'essentiel les éléments décrit dans la cadre du dimensionnement précédemment décrit.

Selon ce deuxième mode de réalisation, on peut considérer que la capsule 30 ne rejoindra le module externe au niveau de la première extrémité à des fins de transmission de données et de recharge de la batterie que toutes les 300 secondes.

En d'autres termes, la batterie doit pouvoir stocker une énergie utile E utile :

[Math 1]

$$E_{utile} = 550 \times 10^{-6} \times 300 = 165mJ$$

Une super-capacité d'une tension maximale de 5,5 V peut être considérée. Seule la moitié de l'énergie stockée dans cette super-capacité est effectivement exploitable. Ainsi, pour un agencement électronique qui fonctionne à une tension de 2,5 V, la valeur C de la capacitance de la super-capacité doit vérifier les relations suivantes :

[Math 2]

$$E_{cap} = \frac{1}{2} C V^2$$

$$C = \frac{2\, E_{cap}}{V^2} = \frac{2 \times 2\ \times E_{utile}}{V^2} = \frac{2 \times 2 \times 165 \times 10^{-3}}{5.5^2} = 21.8\, mF$$

Une super-capacité présentant une capacitance C égale à 22 mF par exemple du type Kemet FYH0H223ZF peut avantageusement en mise en oeuvre.

Ainsi, cette super-capacité, lors d'une phase de recharge, qui peut supporter un courant de l'ordre de 30 mA, se rechargera en un temps $t_{chg}$ :

[Math 3]

$$t_{chg} = \frac{C \times dV}{I_{chg}} = \frac{22 \times 10^{-3} \times (5.5 - 2.7)}{0.030} = 2.05\, s$$

Par ailleurs, les capteurs logés dans la sonde effectuent pendant les 300 secondes de mesures à une fréquence de 1 Hz. Ainsi, l'ensemble de données collectées suit la répartition suivante :

- 2 octets pour le pH ;
- 6 octets pour 3 capteurs de température ;
- 2 octets pour la mesure de dioxygène dissous ;
- 4 octets pour le statut du noeud mobile (niveau de batterie et diagnostic) ;
- 2 octets pour le CRC.

Il en résulte un totale de (2+6+2+4) * 300= 4800 octets.

Il est, dans ce cas de figure, possible de mettre en oeuvre une mémoire RAM d'une capacité de stockage de 20 kB du type STM32L082.

Par ailleurs un protocole NFC pour transmettre l'ensemble des données stockées, permet d'atteindre un minimum 106 kB/s (jusqu'à 424 kB/s), de sorte qu'un temps de l'ordre de 2 seconde est suffisant pour la transmission de l'intégralité des données.

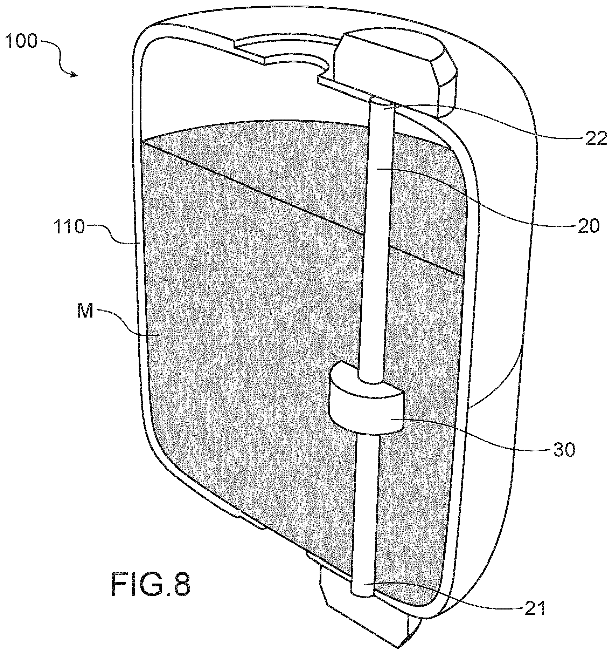

L'invention concerne également un réacteur 100, par exemple un bioréacteur, pourvu d'une enceinte réactionnelle formée par une enveloppe 110 qui délimite un volume interne (figure 8). Le réacteur comprend également le kit 10 décrit ci-avant.

En fonctionnement, le piston aimanté 40, sous l'action des moyens de commande 50, peut effectuer des mouvements de va-et-vient le long du tube et ainsi collecter, avec ses capteurs, des données caractéristiques de l'état du milieu réactionnel M remplissant le volume interne.

Le tube creux 20 fixé à l'enveloppe 110 par ces deux extrémités détermine le trajet effectivement suivi par la capsule 30. Un tel agencement permet d'imposer à la capsule 30 de sonder des zones du milieu réactionnel qui ne le seraient pas si cette dernière n'était pas guidée. Le tube creux peut, selon les besoins, s'étendre entre ses deux extrémités de manière linéaire ou selon toute autre forme susceptible d'être adoptée par un tube.

Le tube creux est par ailleurs agencé de sorte que les ouvertures au niveau de ses extrémités débouchent vers l'extérieur de l'enveloppe. Ces ouvertures autorisent ainsi un accès au volume intérieur du tube creux 20.

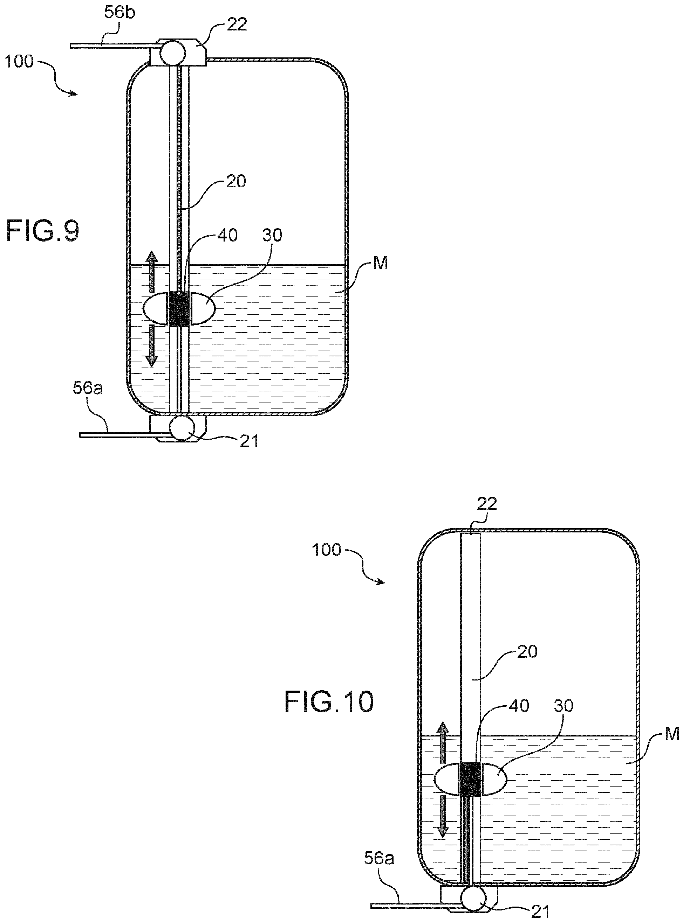

La figure 9 est une représentation schématique du réacteur équipé du kit 10 pourvu des moyens de commande selon la première variante. Selon cette variante, le piston aimanté 40 est commandé par un système de câbles et entraine avec lui la capsule 30 le long du tube creux 20.

Selon un autre aspect illustré à la figure 10, le moyen de commande peut être pourvu d'un unique câble, par exemple uniquement le premier câble 56a. Ainsi, une tension exercée sur ce premier câble 56a permet de diriger la capsule 30 vers la première extrémité 21, tandis qu'un relâchement de ce câble 56a permet, par flottaison de la capsule, de diriger cette dernière vers la deuxième extrémité 22.

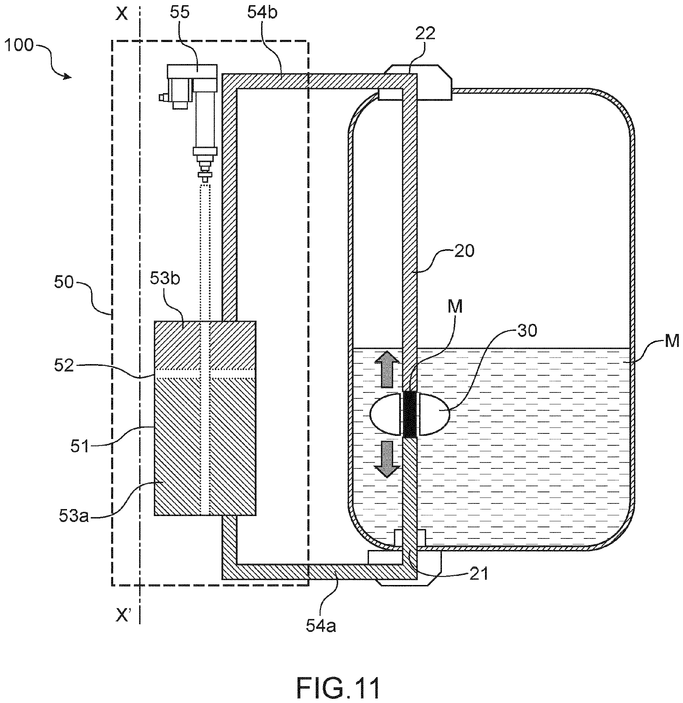

De manière alternative, et tel que représenté à la figure 11, le moyen de commande du kit 10, associés au

**EP 3 967 743 B1**

réacteur 100, peuvent être réalisés selon la deuxième variante. Indépendamment de la variante relative aux moyens de commande, les moyens de réception 60 peuvent, au choix, être réalisés selon l'un ou l'autre du premier mode de réalisation et du deuxième mode de réalisation.

À cet égard, dès lors que le deuxième mode de réalisation est considéré, le module externe 70, tel qu'illustré à la figure 7, est fixé à l'enceinte réactionnelle au niveau de la première extrémité 21 du tube creux 20. Ainsi, lorsque la capsule 30 est en butée contre l'enceinte réactionnelle au niveau de la première extrémité, une communication radio est rendue possible entre les moyens de transmission et les moyens de réception 60. La transmission de données s'effectue au travers de ladite enveloppe.

De manière avantageuse, le module externe 70 peut coiffer le tube creux au niveau de la première extrémité 21.

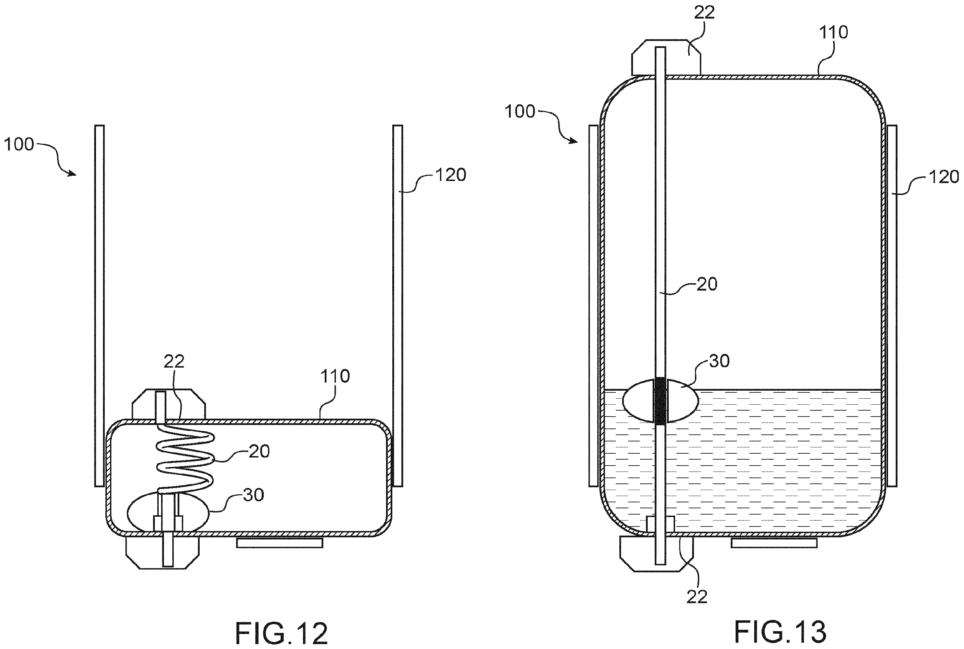

Selon un mode de réalisation particulièrement avantageux, l'enveloppe est formée par un contenant gonflable qui lorsqu'il est gonflé peut être disposé dans une cuve 120. Ce contenant gonflable peut par exemple être un bioréacteur souple à usage unique, classiquement utilisé pour la bio production. Le tube creux 20 peut pour sa part être un tube souple adapté pour adopter une forme d'hélicoïde (par exemple à spire jointives), lorsqu'aucune force n'est appliquée sur ledit tube (figure 12). En d'autres termes, tant que l'enveloppe gonflable est dégonflée, le tube creux conserve sa forme d'hélicoïde.

Le tube creux, fixé par ses extrémités à l'enveloppe gonflable, et alors susceptible d'être étiré pour former un tube qui s'étend linéairement sa première extrémité et sa deuxième extrémité (figure 13). Cette configuration spécifique (contenant gonflable à usage unique avec le tube creux et cellule de mesure intégrés au contenant) permet de garantir la stérilité de l'ensemble du système.

L'invention concerne également un procédé de suivi d'une réaction chimique, biochimique ou biologique mis en oeuvre dans un réacteur 100 selon la présente invention. Le procédé comprend notamment les étapes suivantes :

a) une étape de collecte d'un ensemble de mesures d'au moins un paramètre avec l'au moins un capteur à différentes positions de la capsule le long du tube creux ;
b) une étape d'ajustement de conditions réactionnelles sur la base de l'ensemble des mesures collectées à l'étape a).

Ainsi, la mise en oeuvre du tube creux selon la présente invention permet de préserver l'intégrité de la capsule et des capteurs qu'elle porte.

Par ailleurs, le tube creux qui impose un parcours prédéterminé à la capsule ouvre la voie à la collecte de données dans des zones non couvertes par des capsules libres plongées dans le milieu réactionnel.

En outre, les moyens d'indexation associés aux moyens de commande permettent une localisation simplifiée de la capsule dans le milieu réactionnel.

Enfin, l'agencement proposé limite l'écrantage par le milieu réactionnel du transfert des données collectées.

## RÉFÉRENCES

[1] Lauterbach et al., "Mobile Sensoren fur die Biotechnologie -Ortsunabhangige, miniaturisierte Prozessmessung", C hem. Ing. Tech., 91, No. 12, 1-7, 2019 ;
[2] EP3285070 ;
[3] FR3053165 ;
[4] WO2018172424 ;
[5] P.O Mara et al., "Staying alive! Sensors used for monitoring cell health in bioreactors", Talanta, Volume 176, Pages 130-139, 1 January 2018.

**Revendications**

1. Kit (10) d'analyse et de suivi d'au moins un paramètre d'une réaction chimique ou biochimique ou biologique dans une enceinte réactionnelle, le kit (10) comprenant :

- un tube creux (20) qui s'étend selon deux extrémités dites, respectivement, première extrémité (21) et deuxième extrémité (22), destinées à être fixées sur des parois de l'enceinte réactionnelle ;
- une capsule (30) en liaison glissière avec le tube creux (20), la capsule (30) comprend en outre au moins un capteur (31) configuré pour mesurer l'au moins un paramètre, et des moyens de transmission (34) des données susceptibles d'être mesurées par l'au moins un capteur (31) ;
- un piston magnétique (40) logé à l'intérieur du tube creux (20), coopérant de manière magnétique avec la

9

capsule (30), de sorte qu'un déplacement dudit piston magnétique (40) impose un déplacement à la capsule (30) ;
- un moyen de commande (50) agencé pour commander le déplacement du piston magnétique (40) ;
- des moyens de réception (60) des données susceptibles d'être transmises par les moyens de transmission (34).

2. Kit (10) selon la revendication 1, dans lequel la capsule (30) comprend un canal de guidage (32) traversé par le tube creux (20).

3. Kit (10) selon la revendication 1 ou 2, dans lequel le moyen de commande (50) comprend des moyens d'indexation permettant la détermination de la position de la capsule (30) le long du tube creux (20).

4. Kit (10) selon l'une des revendications 1 à 3, dans lequel le moyen de commande (50) comprend des moyens pneumatiques agencés pour injecter selon l'une et/ou l'autre de la première extrémité (21) et de la deuxième extrémité (22) un fluide permettant d'imposer un déplacement au piston magnétique (40) aimanté.

5. Kit (10) selon la revendication 4, dans lequel les moyens pneumatiques comprennent un réservoir muni d'un piston mobile (52) dont le mouvement contrôlé par un actionneur, notamment un actionneur linéaire, induit l'injection de fluide selon l'une et/ou l'autre de la première extrémité (21) et de la deuxième extrémité (22).

6. Kit (10) selon l'une des revendications 1 à 3, dans lequel le moyen de commande (50) comprend un ou plusieurs câbles agencés pour guider le piston magnétique (40) le long du tube creux (20).

7. Kit (10) selon l'une des revendications 1 à 6, dans lequel le piston magnétique (40) comprend un aimant ou un électroaimant destiné à assurer la coopération magnétique entre le piston magnétique (40) et la capsule (30).

8. Kit (10) selon l'une des revendications 1 à 7, dans lequel le piston magnétique (40) comprend en outre les moyens de réception (60) de sorte que la transmission des données susceptibles d'être mesurées par l'au moins un capteur (31) soient transmises de la capsule (30) vers le piston magnétique (40) selon une transmission radio à travers le tube creux (20).

9. Kit (10) selon l'une des revendications 1 à 8, dans lequel la capsule (30) et le piston magnétique (40) comprennent chacun des moyens d'induction coopérant entre eux et agencés pour alimenter en énergie ladite sonde.

10. Kit (10) selon l'une des revendications 1 à 7, dans lequel la capsule (30) comprend une batterie destinée à assurer l'apport en énergie nécessaire au fonctionnement de l'au moins une sonde et des moyens de transmission (34).

11. Kit (10) selon la revendication 10, dans lequel les moyens de réception (60) sont logés dans un module externe (70) destiné à être disposé hors de l'enceinte réactionnelle et contre la paroi de ladite enceinte.

12. Kit (10) selon l'une des revendications 1 à 11, dans lequel le tube creux (20) forme un hélicoïde, notamment un hélicoïde à spires jointives, et est configuré pour être étiré et s'étendre de manière essentiellement linéaire entre sa première extrémité (21) et sa deuxième extrémité (22).

13. Réacteur (100) qui comprend :

- une enceinte réactionnelle qui comprend une enveloppe (110) délimitant un volume interne ;
- le kit (10) selon l'une des revendications 1 à 12.

14. Réacteur (100) selon la revendication 13, dans lequel le tube creux (20) est disposé dans le volume interne de l'enveloppe (110), et est fixé à cette dernière par ces deux extrémités de sorte que deux ouvertures du tube creux (20) associées à chacune des extrémités débouchent vers l'extérieur de l'enveloppe (110).

15. Procédé de suivi d'une réaction chimique, biochimique ou biologique mis en oeuvre dans un réacteur (100) selon la revendication 13 ou 14, et qui comprend les étapes suivantes :

a) une étape de collecte d'un ensemble de mesures d'au moins un paramètre avec l'au moins un capteur (31) à différentes positions de la capsule (30) le long du tube creux (20) ;
b) une étape d'ajustement de conditions réactionnelles sur la base de l'ensemble des mesures collectées à l'étape a).

**Patentansprüche**

1. Kit (10) zur Analyse und Überwachung mindestens eines Parameters einer chemischen oder biochemischen oder biologischen Reaktion in einer Reaktionskammer, wobei das Kit (10) Folgendes umfasst:

   - ein hohles Rohr (20), das sich entlang zweier Enden erstreckt, die jeweils als erstes Ende (21) und zweites Ende (22) bezeichnet werden und dazu bestimmt sind, an den Wänden der Reaktionskammer befestigt zu werden;
   - eine Kapsel (30) in Gleitverbindung mit dem hohlen Rohr (20), wobei die Kapsel (30) weiter mindestens einen Sensor (31), der so konfiguriert ist, dass er den mindestens einen Parameter misst, und Übertragungsmittel (34) der Daten umfasst, die von dem mindestens einen Sensor (31) gemessen werden können;
   - einen magnetischen Kolben (40), der im Inneren des hohlen Rohrs (20) untergebracht ist und magnetisch mit der Kapsel (30) zusammenwirkt, so dass eine Bewegung des magnetischen Kolbens (40) eine Deplatzierung der Kapsel (30) erzwingt;
   - ein Steuermittel (50), das so angeordnet ist, dass es die Bewegung des magnetischen Kolbens (40) steuert;
   - Empfangsmittel (60) von Daten, die von den Übertragungsmitteln (34) übertragen werden können.

2. Kit (10) nach Anspruch 1, wobei die Kapsel (30) einen Führungskanal (32) umfasst, durch den das hohle Rohr (20) verläuft.

3. Kit (10) nach Anspruch 1 oder 2, wobei das Steuermittel (50) Indexierungsmittel umfasst, die die Bestimmung der Position der Kapsel (30) entlang des hohlen Rohres (20) ermöglichen.

4. Kit (10) nach einem der Ansprüche 1 bis 3, wobei das Steuermittel (50) pneumatische Mittel umfasst, die so angeordnet sind, dass sie entlang des ersten Endes (21) und/oder des zweiten Endes (22) ein Fluid injizieren, was es ermöglicht, dem magnetischen Kolben (40) eine Bewegung aufzuzwingen.

5. Kit (10) nach Anspruch 4, wobei die pneumatischen Mittel einen Behälter mit einem beweglichen Kolben (52) umfassen, dessen Bewegung, die durch einen Aktuator, insbesondere einen linearen Aktuator, gesteuert wird, die Injektion von Fluid gemäß dem einen und/oder dem anderen von dem ersten Ende (21) und dem zweiten Ende (22) induziert.

6. Kit (10) nach einem der Ansprüche 1 bis 3, wobei das Steuerungsmittel (50) ein oder mehrere Kabel umfasst, die so angeordnet sind, dass sie den magnetischen Kolben (40) entlang des hohlen Rohres (20) führen.

7. Kit (10) nach einem der Ansprüche 1 bis 6, wobei der magnetische Kolben (40) einen Magneten oder Elektromagneten umfasst, der dazu bestimmt ist, das magnetische Zusammenwirken zwischen dem magnetischen Kolben (40) und der Kapsel (30) zu gewährleisten.

8. Kit (10) nach einem der Ansprüche 1 bis 7, wobei der magnetische Kolben (40) weiter die Empfangsmittel (60) umfasst, so dass die Übertragung der Daten, die von dem mindestens einen Sensor (31) gemessen werden können, von der Kapsel (30) zu dem magnetischen Kolben (40) gemäß einer Funkübertragung durch das hohle Rohr (20) übertragen werden.

9. Kit (10) nach einem der Ansprüche 1 bis 8, wobei die Kapsel (30) und der magnetische Kolben (40) jeweils Induktionsmittel umfassen, die miteinander zusammenwirken und so angeordnet sind, dass sie die Sonde mit Energie versorgen.

10. Kit (10) nach einem der Ansprüche 1 bis 7, wobei die Kapsel (30) eine Batterie, die dazu bestimmt ist, die für den Betrieb der mindestens einen Sonde erforderliche Energiezufuhr zu gewährleisten, und Übertragungsmittel (34) umfasst.

11. Kit (10) nach Anspruch 10, wobei die Empfangsmittel (60) in einem externen Modul (70) untergebracht sind, das dazu bestimmt ist, außerhalb der Reaktionskammer und an der Wand der Kammer angeordnet zu werden.

12. Kit (10) nach einem der Ansprüche 1 bis 11, wobei das hohle Rohr (20) eine Spirale, insbesondere eine Spirale mit aneinandergrenzenden Windungen, bildet und so konfiguriert ist, dass es gestreckt wird und sich im Wesentlichen linear zwischen seinem ersten Ende (21) und seinem zweiten Ende (22) erstreckt.

13. Reaktor (100), der Folgendes umfasst:

- eine Reaktionskammer, die einen Mantel (110) umfasst, der ein Innenvolumen begrenzt;
- Kit (10) nach einem der Ansprüche 1 bis 12.

14. Reaktor (100) nach Anspruch 13, wobei das hohle Rohr (20) im Innenvolumen des Mantels (110) angeordnet ist und mit beiden Enden an letzterem befestigt ist, so dass zwei Öffnungen des hohlen Rohrs (20), die jedem der Enden zugeordnet sind, zur Außenseite des Mantels (110) hin münden.

15. Verfahren zur Überwachung einer chemischen, biochemischen oder biologischen Reaktion, das in einem Reaktor (100) nach Anspruch 13 oder 14 durchgeführt wird und das die folgenden Schritte umfasst:

a) einen Schritt des Sammelns einer Gesamtheit von Messungen mindestens eines Parameters mit dem mindestens einen Sensor (31) an verschiedenen Positionen der Kapsel (30) entlang des hohlen Rohrs (20);
b) einen Schritt des Einstellens der Reaktionsbedingungen auf der Grundlage der Gesamtheit der Messungen, die in Schritt a) gesammelt wurden.

**Claims**

1. Kit (10) for analyzing and monitoring at least one parameter of a chemical or biochemical or biological reaction in a reaction chamber, the kit (10) comprising:

- a hollow tube (20) that extends according to two end called, respectively, first end (21) and second end (22), intended to be fastened onto walls of the reaction chamber;
- a capsule (30) in a sliding link with the hollow tube (20), the capsule (30) further comprises at least one sensor (31) configured to measure the at least one parameter, and means (34) for transmitting the data that can be measured by the at least one sensor (31);
- a magnetic piston (40) housed inside the hollow tube (20), magnetically cooperating with the capsule (30), so that a movement of said magnetic piston (40) imposes a movement onto the capsule (30);
- a control means (50) arranged to control the movement of the magnetic piston (40);
- reception means (60) for receiving the data which can be transmitted by the transmission means (34).

2. Kit (10) according to claim 1, wherein the capsule (30) comprises a guide channel (32) through which the hollow tube (20) passes.

3. Kit (10) according to claim 1 or 2, wherein the control means (50) comprises indexing means allowing the determination of the position of the capsule (30) along the hollow tube (20).

4. Kit (10) according to one of claims 1 to 3, wherein the control means (50) comprises pneumatic means arranged to inject according to one and/or the other of the first end (21) and the second end (22) a fluid allowing to impose a movement on the magnetized magnetic piston (40).

5. Kit (10) according to claim 4, wherein the pneumatic means comprise a tank provided with a mobile piston (52), the movement of which controlled by an actuator, in particular a linear actuator, induces the injection of fluid according to one and/or the other of the first end (21) and the second end (22).

6. Kit (10) according to one of claims 1 to 3, wherein the control means (50) comprises one or more cables arranged to guide the magnetic piston (40) along the hollow tube (20).

7. Kit (10) according to one of claims 1 to 6, wherein the magnetic piston (40) comprises a magnet or an electromagnet intended to ensure the magnetic cooperation between the magnetic piston (40) and the capsule (30).

8. Kit (10) according to one of claims 1 to 7, wherein the magnetic piston (40) further comprises the reception means (60) so that the transmission of the data that can be measured by the at least one sensor (31) is transmitted from the capsule (30) to the magnetic piston (40) according to a radio transmission through the hollow tube (20).

9. Kit (10) according to one of claims 1 to 8, wherein the capsule (30) and the magnetic piston (40) each comprise induction means cooperating with each other and arranged to supply said probe with energy.

10. Kit (10) according to one of claims 1 to 7, wherein the capsule (30) comprises a battery intended to ensure the provision of energy necessary for the operation of the at least one probe and of the transmission means (34).

11. Kit (10) according to claim 10, wherein the reception means (60) are housed in an external module (70) intended to be disposed outside of the reaction chamber and against the wall of said chamber.

12. Kit (10) according to one of claims 1 to 11, wherein the hollow tube (20) forms a helicoid, in particular a helicoid with touching turns, and is configured to be stretched and extend in a substantially linear manner between its first end (21) and its second end (22).

13. Reactor (100) that comprises:

   - a reaction chamber that comprises an envelope (110) defining an inner volume;
   - the kit (10) according to one of claims 1 to 12.

14. Reactor (100) according to claim 13, wherein the hollow tube (20) is disposed in the inner volume of the envelope (110), and is fastened to the latter by these two ends so that two openings of the hollow tube (20) associated with each of the ends open towards the outside of the envelope (110).

15. Method for monitoring a chemical, biochemical or biological reaction implemented in a reactor (100) according to claim 13 or 14, and which comprises the following steps:

   a) a step of collecting a set of measurements of at least one parameter with the at least one sensor (31) at various positions of the capsule (30) along the hollow tube (20);
   b) a step of adjusting reaction conditions on the basis of the set of the measurements collected in step a).

FIG.1

FIG.2

EP 3 967 743 B1

FIG.3

FIG.4

FIG.5

FIG.6

FIG.7

100

110

M

FIG.8

22

20

30

21

FIG.9

FIG.10

FIG.11

FIG.12

FIG.13

EP 3 967 743 B1

## RÉFÉRENCES CITÉES DANS LA DESCRIPTION

**Documents brevets cités dans la description**

- WO 2021130334 A
- EP 3285070 A
- FR 3053165
- WO 2018172424 A

**Littérature non-brevet citée dans la description**

- **LAUTERBACH et al.** Mobile Sensoren fur die Biotechnologie -Ortsunabhangige, miniaturisierte Prozessmessung. *C hem. Ing. Tech.,* 2019, vol. 91 (12), 1-7
- **P.O MARA et al.** Staying alive! Sensors used for monitoring cell health in bioreactors. *Talanta,* 01 Janvier 2018, vol. 176, 130-139